# EUROPEAN PATENT APPLICATION

(11) **EP 4 643 933 A1**
(43) Date of publication of application: **05.11.2025**
(21) Application number: 24173695.8
(22) Date of filing: 01.05.2024
(51) Int. Cl.: A61N 1/36, A61G 5/00

(54) **IMPROVEMENTS RELATING TO NEUROSTIMULATION**

(71) Applicant: ONWARD Medical N.V., 5611 ZX Eindhoven (NL)
(72) Inventor: Murphy, John, 1005 Lausanne (CH)
(74) Representative: Holmes, Miles Keeton

(57) **Abstract**

A neurostimulator (10) for applying electrical stimulation signals to a user, the neurostimulator having a first mode of operation (14) configured to be independent of a wheelchair, and a second wheelchair mode of operation (16) configured for cooperating with an electronic circuit (22) of a wheelchair.

## Description

Non-limiting aspects of the present disclosure relate to the field of neurostimulation, and ability to adapt neurostimulation to a specific activity or environment of a user.

Broadly speaking, in a first aspect, the disclosure provides a neurostimulator having a first autonomous mode of operation, and a second wheelchair mode of operation configured for co-operating with an electronic circuit of a wheelchair.

One of the important factors in the lives of persons affected by handicap, is their degree of autonomy, that is to say, the ability of a person to complete certain activities either without the need for a caregiver, or with less assistance or intervention of a caregiver. The current aspect of the disclosure has been devised based on an appreciation that, when a user of a neurostimulator is also a wheelchair user, additional functionalities and synergies can be achieved by adapting the operating mode of the neurostimulator when the user is using his or her wheelchair.

Throughout this specification, references to a neurostimulator also extend to mean a component (e.g. component module) of, or for, a neurostimulator, for example, a control module and/or a pulse generator of a neurostimulator. In particular embodiments, the neurostimulator is of a type that is configured to treat a neurological disorder affecting motor control or an autonomic function (e.g. blood pressure, bladder control or continence), but, additionally or alternatively, the neurostimulator may be a type configured to relieve pain. In particular embodiments, the neurostimulator is of a type for applying stimulation to, or in the region of, the spinal cord including, for example, dorsal and/or ventral roots.

Throughout this specification, references to a wheelchair also extend to mean a component (e.g. component module) of, or for, a wheelchair, for example, a module that may be fitted (e.g. retrofitted) to a wheelchair, such as a wheelchair motor, or a motor controller, or a wheelchair controller.

The neurostimulator may be configured to enter, selectively, the first and/or second mode of operation in response to one or more input signals. The input signals may include one or more of (i) manual selection via a user interface of the neurostimulator; (ii) reception of a remote selection signal from a different apparatus; (iii) establishment of electronic communication between the neurostimulator and a wheelchair, for example, establishment of a wired connection and/or a wireless connection.

Broadly speaking, in a second aspect independently of, or optionally in combination with, the first aspect, the disclosure provides a neurostimulator having an electronic communications interface configured for communicating with an electronic circuit of a wheelchair.

Similarly to the first aspect, the current aspect of the disclosure has been devised based on an appreciation that, when a user of a neurostimulator is also a wheelchair user, additional functionalities and synergies can be achieved by exchanging information electronically between the neurostimulator and the wheelchair. This may enhance the ability of a person to complete certain activities either without the need for a caregiver, or with less assistance or intervention of a caregiver.

The communications interface may comprise an interface for wired connection to the wheelchair, and/or an interface for wireless connection to the wheelchair. The communications interface may comprise at least a transmitter, and/or at least a receiver, and preferably comprises a transceiver for multi-direction communication. The communications interface may also comprise a charging function for permitting the or a battery of the neurostimulator to be charged from the usually larger capacity battery of the wheelchair.

The neurostimulator may be configured to transmit information to the wheelchair, for example, status information relating to activity of the neurostimulator, or commands for controlling, at least partly, operation of the wheelchair. In some embodiments, the neurostimulator may be configured to operate independently of the wheelchair, and to transmit status information to the wheelchair. By transmitting status information to the wheelchair, operation of the wheelchair can be adapted depending on stimulation being used, and/or the wheelchair can generate an indication to the user relating to stimulation, for example, an indication on a display unit. Additionally or alternatively, the neurostimulator may comprise or receive sensory input from a brain-computer interface (BCI). The sensory input may include information relating to wheelchair control volition, and such information can be transmitted (e.g. by the neurostimulator) to the wheelchair in the form of commands for commanding operation of the wheelchair.

Additionally or alternatively, the neurostimulator may be configured to receive information from the wheelchair, for example, (i) status information relating to activity and/or configuration of the wheelchair or to the user's interaction with the wheelchair, and/or (ii) commands for controlling, at least partly, operation of the neurostimulator. In some embodiments, operation of the neurostimulator may depend, at least partly, on information received from the wheelchair, to provide hybrid functionality with the wheelchair and/or function aligned with status of the user, or activity or configuration of the wheelchair. Additionally or alternatively, the wheelchair can be used as a controller of the neurostimulator, so that a wheelchair user can input stimulation commands conveniently via a control unit of the wheelchair, instead of having to have to hand, and to operate, an independent controller of the neurostimulator.

Additionally or alternatively, the neurostimulator may be configured to share processing burden (e.g. share responsibility for one or more electronic processing tasks) with the electronic circuit of the wheelchair. Depending on the implementation, a processor of one of the neurostimulator and the wheelchair electronic circuit may have spare capacity that can be used by the other, to complete processing more quickly than one processor alone.

Broadly speaking, in a third aspect independently of, or optionally in combination with, the first and/or second aspect, the disclosure provides a neurostimulator configured to operate in cooperation with an electronic circuit of a wheelchair, to provide neurostimulator functionality dependent on (or aligned with) (i) wheelchair status, for example, wheelchair activity and/or wheelchair configuration, and/or (ii) user interaction information relating to how the wheelchair user interacts with the wheelchair.

Example neurostimulator functionalities include:
(i) Inhibiting generation of stimulation that conflicts (at least potentially) with activity and/or configuration of a wheelchair. For example, while the wheelchair is in movement or when a seat-belt is secured, the neurostimulator may inhibit stimulation that may cause the user to stand. A further example may be, while the wheelchair is in a standing configuration or the user's legs are braced by the wheelchair, the neurostimulator may inhibit stimulation that may cause movement of the user's legs. The neurostimulator may also pause or inhibit certain stimulation until the wheelchair has transmitted information to confirm that the wheelchair activity and/or configuration is non-conflicting with the stimulation.
(ii) Generating stimulation to reduce spasticity and/or to increase blood pressure, in response to receipt of information that that the wheelchair has been commanded to and/or is in the process of changing configuration (e.g. changing between a sitting configuration and a standing configuration, or changing between a sitting configuration and a reclined configuration).
(iii) Generating stimulation to cause the user to adjust his or her sitting position, in response to (i) information that the user has been sitting in the same position on the wheelchair seat for a predetermined period, or (ii) lack of information during a predetermined period that the user has changed position on the seat. Information about the seating position can be obtained by, for example, a pressure sensor incorporated in or otherwise provided at the wheelchair seat.
(iv) Generating stimulation to enhance the user's motor control of his or her hand, in response to receipt of information indicative (directly or indirectly) that the user has difficulty. For example, the wheelchair may be controlled by means of a joystick. Motion of the joystick can be analyzed to detect whether the user seems to have good dexterity, or whether, for example, the user is suffering from hand tremor. In response to such information, stimulation can be controlled (e.g. started, stopped, or varied in amplitude) to enhance the user's voluntary hand control.
(v) Generating stimulation to relieve pain, in response to (i) information that the user has been sitting in the wheelchair for at least a predetermined period of time; and/or (ii) information that the wheelchair is running over relatively rough or non-smooth terrain that may cause discomfort for the user; and (iii) information that the wheelchair has been commanded to change to, or is in, a configuration that the user finds uncomfortable.

Broadly speaking, in a fourth aspect independently of, or optionally in combination with, the first and/or second and/or third aspect, the disclosure provides apparatus comprising a neurostimulator and a cushion (or other support) on which a user can sit or rest during neurostimulation, the neurostimulator being configured to apply stimulation selectively to cause the user to adjust his or her seating position (e.g. seating posture) on the cushion. The cushion may be a seat of or for a wheelchair, or a cushion for other seating.

The neurostimulator may be responsive to (i) reception of information that the user has been sitting in the same position on the cushion for a predetermined period, or (ii) lack of reception of information during a predetermined period that the user has changed position on the cushion. Information about the seating position can be obtained by, for example, a pressure sensor incorporated in or otherwise provided at the cushion. For example, the pressure sensor may detect whether the user is seating generally centrally, or to one side or another (e.g. to the left or to the right).

The stimulation may be applied to induce muscle movement that causes the user to move his or her weight to one side, or centrally.

By causing the user to change or adjust position, at least partly, it is possible to reduce the risk of pressure sores or other discomfort that may result from sitting in the same position for an extended period.

Broadly speaking, in a fifth aspect independently of, or optionally in combination with, the first and/or second and/or third and/or fourth aspect, the disclosure provides a wheelchair (or an electronic circuit therefor), the wheelchair having a first autonomous mode of operation, and a second neurostimulator mode of operation configured for co-operating with a neurostimulator of a wheelchair user.

Similarly to the first aspect, the current aspect of the disclosure has been devised based on an appreciation that, when a wheelchair user also has a neurostimulator, additional functionalities and synergies can be achieved by adapting the operating mode of the wheelchair when the user is using his or her neurostimulator.

The wheelchair (or wheelchair electronic circuit) may be configured to enter, selectively, the first and/or second mode of operation in response to one or more input signals. The input signals may include one or more of (i) manual selection via a user interface of the wheelchair; (ii) reception of a remote selection signal from a different apparatus; (iii) establishment of electronic communication between the wheelchair and a neurostimulator, for example, establishment of a wired connection and/or a wireless connection.

Broadly speaking, in a sixth aspect independently of, or optionally in combination with, the first and/or second and/or third and/or fourth and/or fifth aspect, the disclosure provides a wheelchair (or an electronic circuit therefor) having an electronic communications interface configured for communicating with a neurostimulator.

Similarly to the second aspect, the current aspect of the disclosure has been devised based on an appreciation that, when a wheelchair user also has a neurostimulator, additional functionalities and synergies can be achieved by exchanging information electronically between the wheelchair and the neurostimulator.

The communications interface may comprise an interface for wired connection to the neurostimulator, and/or an interface for wireless connection to the neurostimulator. The communications interface may comprise at least a transmitter, and/or at least a receiver, and preferably comprises a transceiver for multi-direction communication. The communications interface may also comprise a charging function for permitting the or a battery of the neurostimulator to be charged from the usually larger capacity battery of the wheelchair.

The wheelchair may be configured to transmit information to the neurostimulator, for example, (i) status information relating to activity and/or configuration of the wheelchair, and/or status information relating to interaction between the user and the wheelchair, and/or (ii) commands for controlling, at least partly, operation of the neurostimulator. In some embodiments, the wheelchair may be configured to operate independently of the neurostimulator, and to transmit status information to the neurostimulator. By transmitting status information to the neurostimulator, operation of the neurostimulator can be adapted depending on the wheelchair activity and/or configuration and/or status of the wheelchair user. Additionally or alternatively, the wheelchair can be used as a controller of the neurostimulator, so that a wheelchair user can input stimulation commands conveniently via a control unit of the wheelchair, instead of having to have to hand, and to operate, an independent controller of the neurostimulator.

Additionally or alternatively, the wheelchair may be configured to receive information from the neurostimulator, for example, status information relating to activity of the neurostimulator and/or commands for controlling, at least partly, operation of the wheelchair. In some embodiments, operation of the wheelchair may depend, at least partly, on information received from the neurostimulator, to provide hybrid functionality with the neurostimulator.

Additionally or alternatively, the wheelchair (or electronic circuit therefor) may be configured to share processing burden (e.g. share responsibility for one or more electronic processing tasks) with the neurostimulator. Depending on the implementation, a processor of one of the neurostimulator and the wheelchair electronic circuit may have spare capacity that can be used by the other, to complete processing more quickly than one processor alone.

Broadly speaking, in a seventh aspect independently of, or optionally in combination with, the first and/or second and/or third and/or fourth and/or fifth and/or sixth aspect, the disclosure provides an electronic circuit of a wheelchair configured to operate in cooperation with a neurostimulator, to provide wheelchair functionality dependent on (or useful for, or aligned with) neurostimulation.

Example wheelchair functionalities include:
(i) Inhibiting wheelchair activity and/or configuration changes that conflict (at least potentially) with neurostimulation activity. For example, during neurostimulation that may induce leg movements, the wheelchair may be inhibited from motorized movement and/or motorized configuration changes.
(ii) Coordinating wheelchair activity with neurostimulation activity. For example, in response to a user command for wheelchair activity and/or a configuration change, the wheelchair may signal information relating to the command to the neurostimulator. The wheelchair may wait (e.g. at least for a predetermined time period) for a reply signal to be received back from the neurostimulator, indicating for example that no neurostimulation is in progress that would be contrary to the command, and/or that the neurostimulator is coordinating with the command. Upon receipt of a satisfactory reply signal, the wheelchair may execute the command. During execution and/or at the end of execution, the wheelchair may transmit status information to the neurostimulator, to enable the neurostimulator to coordinate neurostimulation activity.
(iii) Determining a time duration during which the user has been seated without changing sitting position, and/or seated in the wheelchair, and/or determining whether the time duration exceeds one or more thresholds. Information about the seating position can be obtained by, for example, a pressure sensor incorporated in or otherwise provided at the wheelchair seat. The wheelchair may transmit information related to the time duration(s) to the neurostimulator to enable the neurostimulator to generate stimulation to cause the user to change slightly his or her sitting position. Additionally of alternatively, the wheelchair may transmit a command (or a request) to the neurostimulator to generate such stimulation.
(iv) During a period when the user is using the wheelchair for physical training or for manual propulsion, monitoring and recording propulsion applied by the user hands to the wheelchair's wheels. For example, the wheelchair can monitor propulsion individually through the left and right wheels, to provide an indication of the user's left and right hands' capabilities (e.g. dexterity and/or strength and/or endurance). The wheelchair can also monitor and record whether stimulation is being applied during the physical training. The recorded information can be reviewed by the user, or by a therapist, as a way of measuring improvement and/or adapting the stimulation program to improve results.
(v) Monitoring the user's hand dexterity, for example, based on how the user manipulates the wheelchair's controls, for example, a joystick. Difficulty in hand control (e.g. hand tremor) can be detected, and this information transmitted to the neurostimulator for processing. Additionally of alternatively, the wheelchair may transmit a command (or a request) to the neurostimulator to generate stimulation to improve voluntary hand control.

The above aspects may be used independently of one another, or any two or more aspects may be used together in combination. In any of the above aspects, the features may be defined in the context of a neurostimulator, a wheelchair, or both together. Any of the above aspects also extends to include a corresponding method of operation, optionally implemented at least partly by software executed by one or more processors.

Although the above presents a summary of some of the main ideas of the present disclosure, protection is not limited only to these aspects. Protection is claimed for any novel feature or idea described herein, and/or illustrated in the drawings, whether or not emphasis has been placed thereon.

Non-limiting embodiments of the disclosure are now described, by way of example only, with reference to the accompanying drawings, in which:
Fig. 1 is a schematic block diagram illustrating certain functionalities of a neurostimulator (Fig. 1a) and a wheelchair (Fig. 1b).
Fig. 2 is a schematic block diagram illustrating electrical component modules and functionalities of a neurostimulator.
Fig. 3 is a schematic diagram illustrating certain mechanical components of a wheelchair;
Fig. 4 is a schematic block diagram illustrating electrical components and functionalities of a combination of a wheelchair and a neurostimulator;
Fig. 5 is a schematic flow diagram illustrating examples of functional interaction between a neurostimulator and a wheelchair.

Referring to the drawings, the embodiments illustrate interactions between a neurostimulator 10 and a wheelchair 12 for a user (not shown). In accordance with some of the principles of the present disclosure, circuitry of the wheelchair 10 and of the neurostimulator 50 can communicate to provide interactions and additional functionality not previously possible with each system independently. While some of the following includes a description of many elements of the neurostimulator 10 and wheelchair 12 that may be useful for interaction together, it will be appreciated that in some embodiments, and whether or not explicitly elicited below, some elements or features may be omitted depending on the functionality desired to be implemented. Not all features are necessary, and no features are to be regarded as essential to all embodiments.

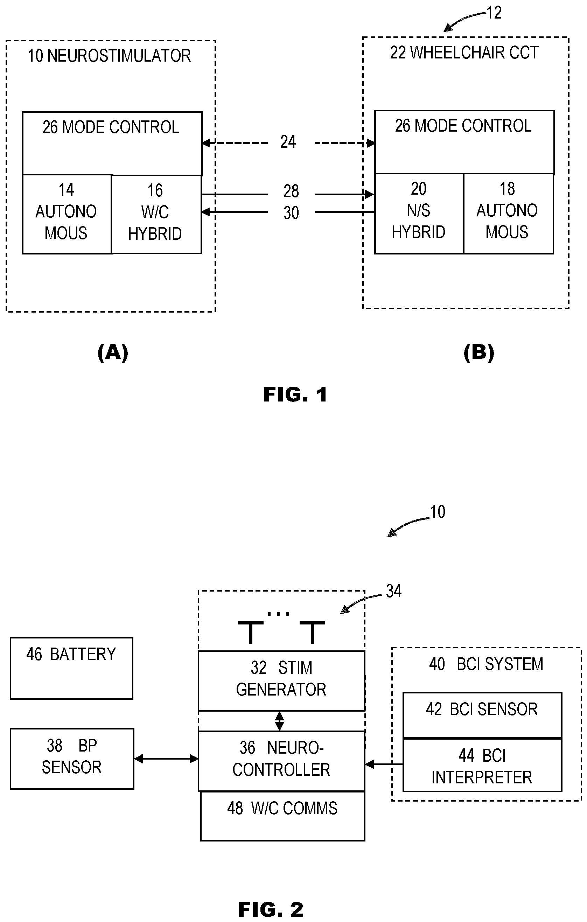

Referring to Fig. 1a, the neurostimulator 10 may have (at least) two modes of operation, a first mode 14 being an autonomous mode (independent of or unrelated to the user being a wheelchair user), and the second mode 16 being a wheelchair mode, or hybrid mode, for interaction with an electronic circuit 22 of or for a wheelchair, also referred to herein as a wheelchair circuit 22. Similarly, referring to Fig. 1b, a wheelchair circuit 12 may have (at least) two modes of operation, a first mode 18 being an autonomous mode (unrelated to any neurostimulation), and the second mode 20 being a neurostimulation mode, or hybrid mode, for interaction with a neurostimulator 10.

The selection of the different modes 14-20 may be made manually, or in response to external input signals 24 received by a respective mode selection module 26. In the illustrated example, the mode selection module 26 may be responsive to a communications connection (wired and/or wireless) being established between the neurostimulator 10 and the wheelchair circuit 22.

The neurostimulator 10 is configured, when in the hybrid mode, to transmit information 28 to the wheelchair circuit 22 and/or to receive information 30 from the wheelchair circuit 22. The wheelchair circuit 22 is configured, when in the hybrid mode, to receive information 28 from the neurostimulator 10 and/or to transmit information 30 to the neurostimulator 10.

In the above example, the transmission and reception of information 28 and 30 between the neurostimulator 10 and the wheelchair circuit 22 is responsive to the operating mode of the elements, to provide versatile operation according to the most appropriate mode. In other examples which do not implement distinct operative modes, the transmission and reception of information 28 and 30 need not be so dependent.

Referring to Fig. 2, the neurostimulator 10 may optionally be a portable unit, independent of the wheelchair 12, and configured to apply electrical stimulation signals to the body to treat a neurological disorder (or another ailment or discomfort). Stimulation can include stimulation of the spinal cord, and/or stimulation of dorsal roots of the spinal cord, and/or deep brain stimulation, and/or functional electrical stimulation.

The neurostimulator 10 generally comprises a generator 32 for generating electrical stimulation signals, and at least one, optionally a plurality, of electrodes 34 by which the stimulation signals are applied to the user. In some examples, at least some of the electrodes may be implemented together in an electrode assembly (e.g. an assembly of 2, 4, 6, 8, 10, 12, 14, 16, 18, 20, 22, 24, 26, 28, 30, 32, 34 electrodes in a regular or irregular array). Additionally or alternatively, at least some of the electrodes may be distinct or separate from one another.

At least one (optionally at least some) of the electrodes 34 may be configured to be carried or worn outside the body for transdermal stimulation, and/or at least one (optionally at least some) of the electrodes 34 may be configured to be implantable (at least partly or wholly) within the body, for epidural or deeper stimulation of body tissues. Especially for electrodes that are implanted, the generator 32 may optionally also be configured to be implantable within the body and connected by implantable leads.

The neurostimulator 10 may further comprise a controller 36 for controlling operation of the generator 32, for example, for setting parameters of the generator 32 that may determine stimulation characteristics. The parameters may include any of: pulse timing; pulse sequence timing; stimulation frequency; stimulation amplitude (voltage and/or current); burst-characteristics; carrier-frequency characteristics; polarity (e.g. mono-phase polarity or bi-phase polarity); different pulse sequences; selection of electrodes to which the stimulation signals are applied to target different neural circuits. By varying the parameters, different therapeutic effects can be achieved. Examples include increase in blood pressure, reduction in tremor (for example, if the user is affected by Parkinsons disease), and at least partial activation of selected muscles or muscle groups for motor control, for example, of the hands, arms, legs, feet, and torso. Stimulation can be applied at an amplitude to directly control muscles to force involuntary movement, and/or stimulation can be applied at a lesser amplitude to aid the user in activating neurological circuits to better exercise voluntary motor control. Since neurological disorders can affect users differently, selection of certain parameters by a physician can also be an ongoing, iterative exercise, and the therapeutic effects can be progressive as the user's brain becomes trained to exploit neural circuits enabled by stimulation.

The controller 36 may be integrated into the same unit as the generator 32, or may be configured as a separate unit. In the case that the generator 32 is configured to be implantable, the controller 36 may be a separate unit, optionally not designed to be implantable. The controller 36 may be configured to communicate wirelessly with the generator 32, and optionally also to provide a charging signal for inductive charging of the generator 32. In the case that the generator 32 is not configured to be implantable, the controller 36 may be conveniently housed in the same physical unit as the generator 32, and the circuitries optionally integrated together as a single circuit instead of being distinct.

In some (but not all) embodiments, the neurostimulator 10 may also comprise, and/or receive information from, a blood-pressure sensor 38. Various implementations of blood pressure sensor are envisaged. For example, the blood pressure sensor 38 may comprise an inflatable cuff worn by the user, the cuff being inflated occasionally (e.g. periodically) to enable blood pressure measurements to be derived. Alternatively, the blood pressure sensor 38 may determine blood pressure indirectly from a measurement of one or other parameters, for example, based on an optical or ultrasonic measurement of blood flow in a blood vessel or blood perfusion in body tissue. The signal from the blood pressure sensor 38 may be used as a control input (e.g. closed-loop control input), for triggering stimulation. For example, in case blood pressure is determined to have fallen below a predetermined threshold, the neurostimulator may be operative to generate stimulation to increase and/or stabilize blood pressure.

In some (but not all) embodiments, a so-called brain-computer interface (BCI) 40 may be provided as part of, or associated with, the neurostimulator 10. The BCI 10 may generally comprise a BCI sensor 42 provided at (or for provision at) the user's brain, for sensing electrical signals from within the brain. The BCI sensor 42 may be (e.g. configured for) implanted at least partly within the user's head, in or adjacent to brain tissues. The BCI further comprises a BCI interpreter module 44 for interpreting the signals sensed by the sensor 42 at least to a degree that is useful for detecting volition of the user to perform a certain activity. The BCI interpreter 44 may be implemented at least partly as a distinct processing module, for example, associated with the BCI sensor 42, or it may be implemented at least partly in (or associated with) the controller 36 of the neurostimulator 10.

In the current example, the neurostimulator 10 is operable independently of the wheelchair 12 to provide neurostimulation therapy whether or not the user is using the wheelchair 12. A single battery (e.g. rechargeable battery) 46 of the neurostimulator 10 is illustrated schematically, although it will be appreciated that multiple batteries may be implemented for powering different units or modules of the neurostimulator.

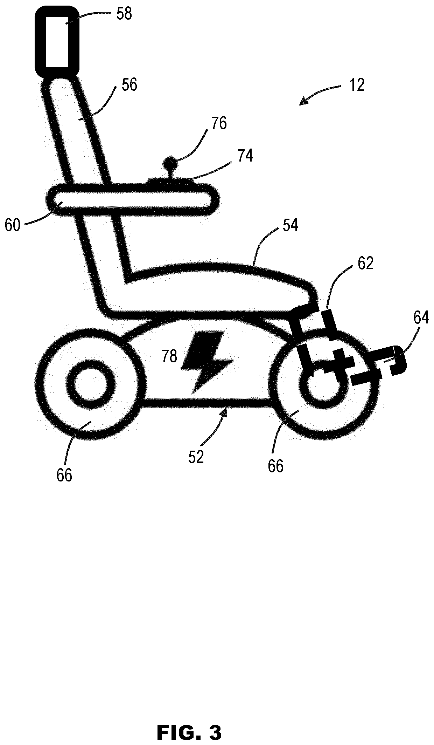

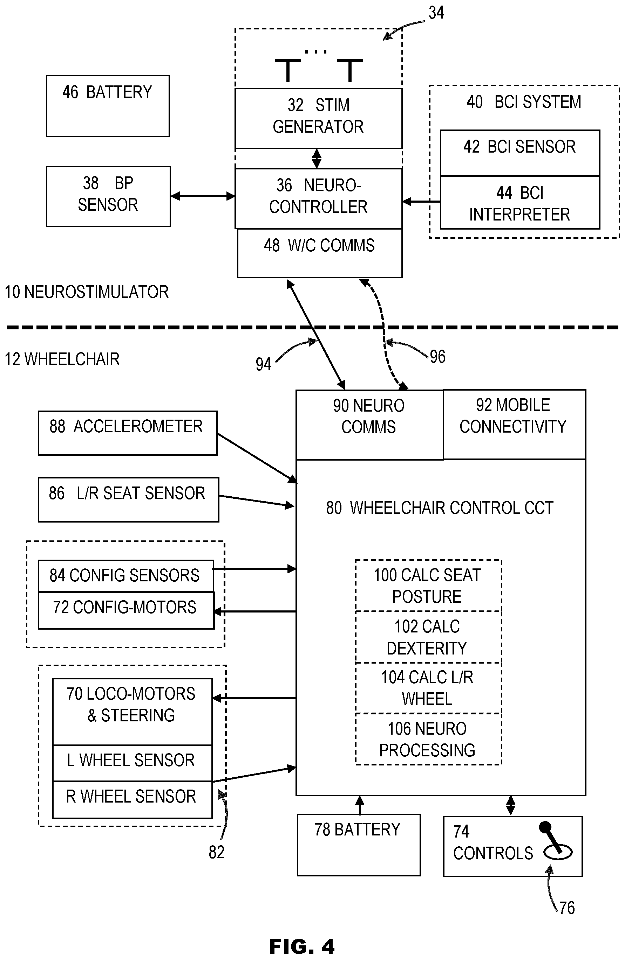

Referring to Figs. 3 and 4, the wheelchair 12 itself may generally comprise a wheeled carriage 52, supporting one or more of: a seat 54, a back-rest 56, a head-rest 58, at least one arm-rest 60 (optionally a pair), at least one leg-rest 62 (optionally a pair), and at least one foot-rest 64 (optionally a pair). Depending on the design of wheelchair, one or more of the above elements may be omitted. The elements may have a generally fixed relation (to define a fixed, seated posture for the wheelchair user), or one or more of the elements may be movable (e.g. to define multiple possible configurations to support multiple postures of the wheelchair user). For example, the wheelchair may have, in addition to a seated configuration, a standing configuration in which one or more of the seat 54, back-rest 56 and/or head-rest 58 are moved into an upright profile, to support the wheelchair user standing upright. Additionally or alternatively, the wheelchair 12 may have a reclined configuration, in which one or more of the back-rest 56, head-rest 58, leg rest 52 and foot rest 54 are moved into a reclined profile, to support the wheelchair user reclining or lying (e.g. lying flat). In some examples, some of the parts 54-64 may be connected by articulating joints or supported by an articulating frame, to facilitate movement between the different configurations.

The carriage 12 further comprises multiple wheels 66. Although not limiting, the number of wheels may typically be four, five or six. The wheels 66 may be of generally the same size or of different sizes. For example, two opposed wheels may be relatively large (e.g. compared to others), and referred to as principal wheels. Other wheels 66 may be smaller, may lead and/or trail the principal wheels, and may be actively or passively steerable.

In some (but not necessarily all) examples, the wheelchair 12 may be motorized, e.g. permanently or temporarily. One or more locomotive motors 70 (Fig. 4) may be provided for wheelchair locomotion. Steering may be accomplished by differential drive between the wheels, and/or by an active steering mechanism for, for example, directing the leading or trailing wheels. The motor(s) 70 may be integrated as part of the wheelchair 12, or they be part of a locomotion module (not shown) that may be retrofitted, permanently or temporarily, to the wheelchair 12.

Additionally or alternatively, one or more configuration motors 72 (Fig. 4) may be provided for driving different parts 54-68, or their relative orientations, to change the configuration of the wheel chair (e.g. between the seated and standing configurations, and/or between the seated and reclining configurations).

The wheelchair 12 may be equipped with a control unit (or console) 74 for permitting the wheelchair user to control operation of the wheelchair 10. The control unit 74 may, for example, be carried on an arm rest 60. The control unit 74 may optionally comprise a joystick 76 and/or other manual controls (e.g. physical button switches and/or touch-sensitive display switches - not shown).

The wheelchair 12 can further comprise a rechargeable battery 78 and control circuitry 80 for controlling operation of the wheelchair 12, in particular by controlling generation of drive signals to the locomotive motors 70 and/or to the configuration motors 72. The control circuitry, optionally in addition to other modules of Fig. 4, may be or correspond to the wheelchair circuit 22 described above. In addition to receiving an input signal from the control unit 74, the circuitry 80 may receive inputs from one or more other sensors 82-88 of the wheelchair. Such sensors may optionally include one or more of:
- wheel sensors 82 for sensing rotation and/or torque and/or force applied via the wheels 26. Whether or not the wheelchair is motorized, such sensors are especially useful for measuring the force that a user may manually apply by hand to propel the wheelchair. Each wheel may include a hand ring or circumferential bar that the wheelchair user can grip to apply torque. A sensor may be provided between the ring and the wheel, or between the wheel and an axle (or drive), to measure that force. Distinct sensors "L" and "R" may measure the torque applied via the left and right wheels, by the wheelchair user's left and right hands.
- Configuration sensors 84 for sensing the operative position of one or more of the parts 44-68, to provide input about the current configuration of the wheelchair. One or more further configuration sensors 84 may sense the presence and/or the operative position of one or more additional parts, not shown. Such additional parts may, for example, include (i) a manually fittable and/or manually movable leg brace that may be used to brace the wheelchair user's legs when standing (ii) a manually fittable and/or manually movable seat belt or seat brace for securing the wheelchair user safely to the wheelchair.
- A seat sensor 86 for sensing the presence of the wheelchair user on the seat 44, for example, by measuring weight or pressure on the seat. Optionally, the seat sensor 86 can sense the seating position, for example, whether the user is applying more weight to the left or to the right. For example, the seat sensor 86 may comprises two pressure sensors, positioned to the left and right, respectively of the seat middle, or a balance sensor to sensing weight distribution between left and right.
- One or more accelerometers 88 (e.g. a three-dimensional accelerometer) for sensing the orientation of the wheelchair, and physical accelerations, to provide an indication of the wheelchair state.

In accordance with some of the principles of the present disclosure, the circuitries of the neurostimulator 10 and the wheelchair 12 can communicate to provide additional interactions not previously possible with each system independently. The wheelchair circuit 22 comprises or is coupled to a communications interface 90. The neurostimulator 10 comprises or is coupled to a communications interface 48 for communicating with the interface 90. The interfaces 48 and 90 may be connectable together via a wired connection 94 and/or a wireless connection 96. A wired connection 94 can facilitate high-speed information exchange as well as efficient transfer of power (e.g. for keeping the neurostimulator battery(ies) 46 charged from larger capacity wheelchair battery 78). A wireless connection 96 removes the need for attaching a physical connector between the neurostimulator 10 and the wheelchair 12, but with more complications for information and power transfer. In some embodiments both wireless and wired connections may be implemented, for example, wireless for information exchange and wired (when connected) for power transfer (and optionally also information exchange).

The wheelchair circuit 22 may optionally further comprise a further radio communications interface 92, for example, a cellular or cellular data transceiver, for enabling mobile communications, for example, with internet services, or a remote processor, or server, or a remote monitor for the wheelchair or wheelchair user.

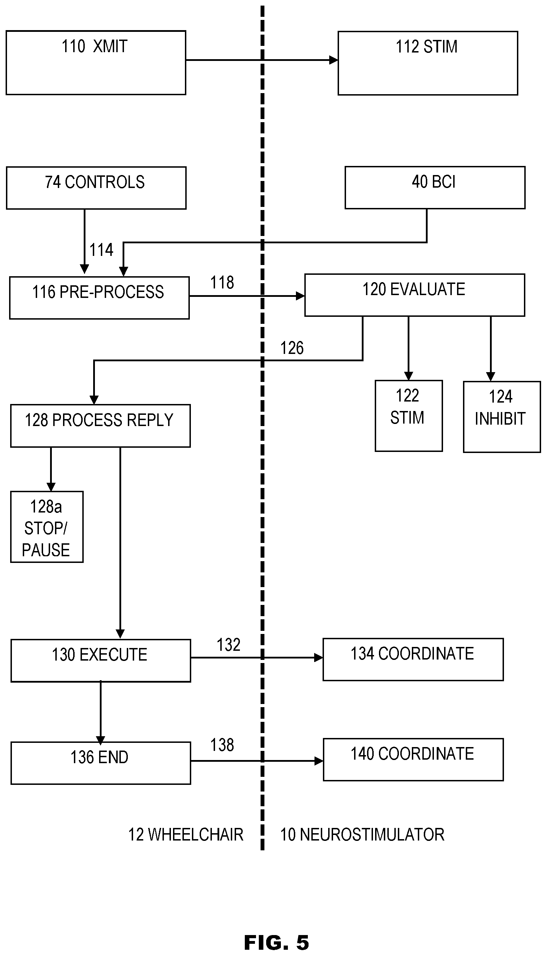

To assist in interactions with the neurostimulator 10, the control circuit 80 may optionally comprises a processor that is configured to implement one or more of the following functional modules:
- A module 100 for monitoring the user's sitting position based on a signal from the seat sensor 86. The module 100 can determine for how long the user has been sitting in the same position, and/or whether that time period exceeds a predetermined seat threshold. Referring also to Fig. 5, at step 110, such information is or can be transmitted to the neurostimulator 10 and, responsive to the information received, at step 112, the neurostimulator 10 can generate electrical stimulation signals to cause the user to move at least slightly (e.g. involuntarily), in order to adjust his or her seating position, and reduce risk of sores, discomfort or other injuries caused by stationary sitting for too long. Although this functionality is illustrated here in an example of a wheelchair seat, the same functionality may be implemented with any seat, cushion, recliner or mattress for the user, provided with a sensor, in order to reduce risk of sores or other discomfort or injuries caused by prolonged resting in the same position without movement.
- A module 102 for monitoring the user's dexterity based on a signal received from, for example, the joystick 76. Should the user experience difficulty in manipulating the joystick, or suffer from hand tremors, such a condition can be detected based on inputs from the joystick 76. Referring also to Fig. 5, at step 110, such information is or can be transmitted to the neurostimulator 10 and, responsive to the information received, at step 112, the neurostimulator 10 can generate electrical stimulation signals to assist the user's voluntary hand control.
- A module 104 for monitoring and recording physical exercise of the user, by manual propulsion of the wheels, based on the signal or signals from the wheel sensor(s) 82. The ability of the user to turn propel the wheels can provide information indicating the user's strength, dexterity, and endurance. By recording such information, the user or a therapist can observe the user's abilities and progress as a result of physical exercise. In a preferred embodiment, such exercise is carried out in combination with neurostimulation. The module 104 is optionally configured to record stimulation information (e.g. whether stimulation used, and stimulation program) transmitted to the wheelchair circuit 80 by the neurostimulator 10.
- A module 106 for carrying out some of the processing burden of the neurostimulator 10 when the wheelchair 12 is used in combination with the neurostimulator 10. The circuit 80 of the wheelchair may have greater processing capability, and/or be less constrained by power consumption limitations, than the neurostimulator 10. The module 106 may also enable information obtained from or for the neurostimulator 10 to be transmitted remotely, by means of the wheelchair's mobile connectivity 92.

Fig. 5 illustrates additional interactions envisaged between the neurostimulator 10 and the wheelchair 12 (or wheelchair circuit 22), in response to a command signal 114 (which may be or include a configuration status signal) at the wheelchair 12. The command signal 114 may be derived from the control unit 74, and/or from user volition information decoded from the BCI system 40 and transmitted to the wheelchair circuit 22. The command signal may, for example, correspond to desired locomotion of the wheelchair, or to a desired change in wheelchair configuration, or merely to wheelchair configuration status.

At step 116, the wheelchair circuit 22 processes the command signal and transmits (at 118) information about the command to the neurostimulator 10. At step 120, the neurostimulator determines whether there is any conflict between the wheelchair command and stimulation.

Also at step 120, the neurostimulator determines whether certain stimulation should selectively be initiated to align with the command, or selectively inhibited to align with the command. For example, if the command is a change in wheelchair configuration (e.g. between sitting and standing), the neurostimulator may generate electronic stimulation signals (step 122), for example, to increase blood pressure, or to reduce spasticity. Additionally, the neurostimulator 10 may also process the information as an input to help interpret blood pressure from the blood pressure sensor 38. Information relating to a change in posture of the user may be used as a way of detecting, more quickly and reliably, a drop in blood pressure than from the blood pressure sensor 38 alone. This may particularly be the case if, for example, the sensor 38 measures blood pressure indirectly from another sensed parameter, and relies on presumption that blood pressure is generally relatively steady or stable once a baseline pressure is established. Warning of a change in posture can be used as a corrective factor.

Still at step 120, if the command is wheelchair movement (or indicates a wheelchair configuration that limits leg movement), the neurostimulator may inhibit (step 124) stimulation that might be in conflict, for example, stimulation that may cause, directly or indirectly, unwanted leg muscle movements.

Also at step 120, the neurostimulator 10 transmits a reply or acknowledgement 126 to the wheelchair circuit 22, indicative of the neurostimulator status, and/or whether the command is safe to execute.

At step 128, the wheelchair circuit processes the reply/acknowledgement. If the reply/acknowledgement is not satisfactory (e.g. conflicts with the command), at step 128a, the command (and optionally certain wheelchair functionality that may conflict with current stimulation activity of the neurostimulator 10) may be inhibited or paused until the conflict ends.

If satisfactory, the command is executed by the wheelchair control circuit 80 at step 130. Also at step 130, status information 132 indicative of command execution is transmitted to the neurostimulator 10. At step 134, responsive to the status information 132, the neurostimulator 10 may selectively initiate, continue, or discontinue generation of stimulation signals, to coordinate with execution of the command.

At step 136, similarly to step 130, when execution of the wheelchair command ends, status information 138 indicative of command termination is transmitted to the neurostimulator 10. At step 140, responsive to the status information 138, the neurostimulator 10 may selectively initiate, continue, or discontinue generation of stimulation signals.

The above are merely examples of interactions and additional functionalities obtainable by communication between a neurostimulator and a wheelchair for a user. It will also be appreciated that, while some circuit modules and functions have been described as being incorporated in the neurostimulator or in the wheelchair circuit, the disclosure is not so limited. In order examples, circuit modules and functions may be swapped between the neurostimulator and the wheelchair to suit the desired implementation. Many other modifications and equivalents can also be used within the scope and principles of the disclosure.

## Claims

1. A neurostimulator for applying electrical stimulation signals to a user, the neurostimulator having a first mode of operation configured to be independent of a wheelchair, and a second wheelchair mode of operation configured for cooperating with an electronic circuit of a wheelchair.

2. The neurostimulator of claim 1, configured to enter selectively the first and/or second mode of operation in response to an input signal, the input signal selected from (i) manual selection through a user interface of the neurostimulator; (ii) reception of a remote selection signal from a different apparatus; and/or (iii) establishment of electronic communication between the neurostimulator and a wheelchair.

3. A neurostimulator, optionally according to any preceding claim, the neurostimulator for applying electrical stimulation signals to a user and comprising an electronic communications interface configured for communicating with an electronic circuit of a wheelchair, the interface being configured to transmit information to the wheelchair and to receive information from the wheelchair.

4. The neurostimulator according to claim 3, wherein the interface is configured for wireless and/or wired communication with a wheelchair.

5. A neurostimulator according to claim 3 or 4, wherein the neurostimulator is configured to process information received from the wheelchair as one or more of:
(a) status information relating to wheelchair activity;
(b) status information relating wheelchair configuration;
(c) status information relating to user interaction with the wheelchair;
(d) a command for controlling, at least partly, operation of the neurostimulator;

6. The neurostimulator according to claim 3, 4 or 5, wherein the neurostimulator is configured to transmit to the wheelchair information selected from:
a. status information relating to neurostimulation status;
b. status information relating to a stimulation program generated or to be generated by the neurostimulator;
c. information derived from a brain-computer-interface;
d. an acknowledgement responsive to information received from the wheelchair;
e. an information response to information received from the wheelchair.

7. A neurostimulator, optionally according to any preceding claim, the neurostimulator for applying electrical stimulation signals to a user and configured to operate in cooperation with an electronic circuit of a wheelchair, to provide neurostimulator function dependent on one or more of:
a. wheelchair activity
b. wheelchair configuration
c. user interaction information relating to interaction of the user with the wheelchair.

8. The neurostimulator of any preceding claim, wherein the neurostimulator is configured to generate electrical stimulation signals in response to information received from the wheelchair relating to a change in wheelchair configuration, optionally a change between a sitting configuration and a standing configuration and/or between a sitting configuration and a reclined configuration.

9. The neurostimulator of claim 8, wherein the electrical stimulation signals are generated to functionally:
a. Increase blood pressure of a user; and/or
b. Reduce user spasticity.

10. The neurostimulator of any preceding claim, wherein the neurostimulator is configured to inhibit generation of stimulation in response to information received from the wheelchair.

11. The neurostimulator of any preceding claim, configured to generate stimulation to cause a user to adjust his or her sitting position in response to information received from the wheelchair indicative of (i) the user not having changed his or her sitting position during a predetermined period of time, and/or (ii) a command from the wheelchair based on the user not having changed his or her sitting position.

12. The neurostimulator according to any preceding claim, configured to generate stimulation in response to information received from the wheelchair about dexterity of the user, optionally wherein the electrical stimulation signals are generated to functionally assist the user's hand control.

13. The neurostimulator according to any preceding claim, wherein the neurostimulator is configured for stimulation of the spinal cord.

14. The neurostimulator according to any preceding claim, wherein the neurostimulator comprises a pulse generator selected from: (i) an implantable pulse generator optionally for epidermal stimulation, and (ii) a non-implantable pulse generator optionally for transcutaneous stimulation.

15. Apparatus comprising a neurostimulator according to any preceding claim, and a brain-computer interface configured for communication directly or indirectly with the neurostimulator for providing signals from the brain indicative of volition and/or from which volition is derivable.
